# EUROPÄISCHE PATENTSCHRIFT

(11) **EP 2 826 549 B1**
(45) Veröffentlichungstag und Bekanntmachung des Hinweises auf die Patenterteilung: **06.07.2016**
(21) Anmeldenummer: 13003588.4
(22) Anmeldetag: 17.07.2013
(51) Int. Cl.: B01F 7/02, B01F 7/00, B01F 15/00, C12M 1/06, C12M 1/107

(54) **Vorrichtung zum Durchmischen des Inhalts von Substratbehältern**
Device for mixing the content of substrate containers
Dispositif de mélange du contenu de récipients de substrats

(43) Veröffentlichungstag der Anmeldung: 21.01.2015
(73) Patentinhaber: BHKW Johann Hochreiter Biogas Planung Beratung GmbH, 83530 Schnaitsee (DE)
(72) Erfinder: Hochreiter, Johann, D-83530 Schnaitsee (DE)

(56) Entgegenhaltungen:
- EP-A2- 0 123 790
- AT-A4- 506 211
- DE-U1-202004 004 101
- DE-U1-202004 005 331
- DE-U1-202004 012 236
- FR-A1- 2 336 168
- GB-A- 890 215

## Beschreibung

Die vorliegende Erfindung bezieht sich auf eine Vorrichtung zum Durchmischen des Inhalts von Substratbehältern gemäß dem Oberbegriff des Anspruchs 1.

Rührwerke für Biogasanlagen sind bereits bekannt. So ist in der DE 102 60 972 B4 eine Vorrichtung zum Durchmischen von Flüssigmist in einem senkrecht stehenden Vorratsbehälter beschrieben, die sich dadurch auszeichnet, dass eine Welle mit Rührelementen radial in den senkrecht stehenden, zylindrischen Vorratsbehälter hineinragt, mittels einer Antriebsvorrichtung drehbar ist und dass die Rührelemente als schaufelförmige Paddel so ausgebildet sind, dass sie in bestimmten Rotationspositionen einen Durchbruch in einer Abdeckung des Vorratsbehälters durchragen, und dass der Durchbruch mittels einer Haube hermetisch verschließbar ist.

Ferner ist in der DE 10 2008 006 813 A1 eine Rührvorrichtung für Biogasfermenter gezeigt und beschrieben, die zur Wartung oder Reparatur problemlos ohne Veränderung des Füllstands und ohne wesentliche Störung des normalen Betriebsablaufs ausbzw. eingebaut werden kann. Dazu befindet sich die Rührvorrichtung in einem senkrecht stehenden Schacht, der von oben her in den Fermenterkessel hineinragt und im unteren Teil eine Austrittsöffnung hat. Die Austrittsöffnung liegt dabei etwas unterhalb der Füllstandshöhe, so dass ein gasdichter Abschluss entsteht. Der Schacht mit der Rührvorrichtung kann auch an der Außenseite des Fermenters angebracht sein.

Außerdem ist eine Biogasanlage mit einem Rührwerk aus der DE 20 2006 004 982 U1 bekannt. Dort umfasst das Rührwerk zumindest einen Antrieb und ein an der Wand des Fermentationsbehälters befestigtes wandseitiges Rührwellenlager, ein weiteres, sich am Boden abstützendes fermenterseitiges Rührwellenlager und zumindest eine Rührwelle mit Rührblättern. Dabei erstreckt sich die Rührwelle parallel zum Boden des Fermentationsbehälters in Richtung der Hauptachse des Fermentationsbehälters.

Außerdem ist aus der DE 20 2006 020 195 U1 ein Rührwerk für einen Fermenter bekannt, bei dem mehrere vertikal angeordnete Rührwellen Rührpaddel tragen und über einen Antriebsmechanismus zum Antrieb der Rührwellen verfügen. Am Boden des Fermenters befinden sich Zentrierlager zum zentrieren der vertikal angeordneten Rührwellen.

In der DE 20 2004 012 236 U1 ist ein Rührwerk für Biogasfermenter gezeigt und beschrieben, bei dem ein schräg ins innere des Fermentationsbehälters ragendes Rührwerk zwei Rührschaufeln auf seiner Rührwelle trägt. Die Rührschaufeln sind Propellerartig ausgebildet und in gleicher Ausrichtung auf der Rührwelle positioniert. Ihre Rotationskreise überschneiden sich in Bezug auf das Flüssigkeitsniveau im Fermentationsbehälter nicht. Kern des dort beschriebenen Gegenstands ist die speziell ausgestaltete Fermenterseitige Lagerkonsole.

*In der* EP 0 123 790 A2 *ist eine Rührvorrichtung entsprechend dem Oberbegriff des Anspruchs 1, zum Durchmischen von Flüssigkeiten für einen Behälter gezeigt und beschrieben. Die Rührvorrichtung ist schrägstehend angeordnet und weist zwei übereinander liegende Rührer auf. Die Rührflügel sind bei dieser Vorrichtung propellerartig ausgebildet und weisen ein tragflächenartiges Profil auf.*

Der vorliegenden Erfindung liegt die Aufgabe zu Grunde, eine Vorrichtung zum Durchmischen des Inhalts von Substratbehältern, insbesondere bei Biogasanlagen zu schaffen, die über eine hohe Leistungsfähigkeit verfügt, sehr gut an die wechselnden Bedingungen beim Betrieb der Biogasanlage anpassbar ist und bei der Wartung geringe Störungen des Betriebs verursacht.

Diese Aufgabe wird von einer Vorrichtung mit den Merkmalen des Anspruchs 1 gelöst. Vorteilhafte Ausgestaltungen der Vorrichtung sind den abhängigen Ansprüchen zu entnehmen.

Die Vorteile der erfindungsgemäßen Vorrichtung für Biogasanlagen liegen in seiner großen Leistungsfähigkeit, ihrer Stabilität und ihrer Wartungsfreundlichkeit.

In dem Substratbehälter ist, ein Rührwerk installiert, dessen Rührschaufeln in ihrer Länge, ihrer axialen Position auf einer Welle des Rührwerks und der Einbauwinkel der Welle so gewählt sind, dass sich ihre Rotationskreise in Bezug auf das Flüssigkeitsniveau im Substratsbehälter überlagern und bei Rotation des Rührwerks eine rotierende Woge des Substrats im Substratbehälter erzeugen.

*Entsprechend der Erfindung ist eine Vorrichtung zum Durchmischen des Inhalts von Substratbehältern ausgestaltet, wenn der Substratbehälter eine umlaufende Wand und einen Boden umfasst, wobei sich die umlaufende Wand an einer vertikalen Hauptachse orientiert, wenn die Vorrichtung zum Durchmischen des Inhalts von Substratbehältern ein Rührwerk aufweist und das Rührwerk eine Welle umfasst, die unter einem von der Vertikalen und Horizontalen abweichenden Einbauwinkel in den Substratbehälter hineinragt, und wenn das Rührwerk wenigstens zwei Rührschaufeln umfasst, die sich jeweils radial beidseits der Welle erstrecken und zueinander verdreht sowie in Längserstreckung der Welle voneinander beabstandet auf der Welle angeordnet sind, wobei die Länge der Rührschaufeln, ihre axiale Position und der Einbauwinkel der Welle so gewählt sind, dass sich ihre Rotationskreise in Bezug auf das Flüssigkeitsniveau im Substratsbehälter überlagern, und wenn die Rührschaufeln bis in ihre Endbereiche ein sich radial zur Welle erstreckendes Rechteckprofil aufweisen, die Endbereiche gegenüber der Erstreckungsrichtung des Profils jedoch Abwinklungen aufweisen.*

*Eine Vorrichtung ist dann besonders vorteilhaft, wenn die Rührschaufeln ein prismatisches Profil mit Schiffsbugartiger Schneide aufweisen.*

Bei der Vorrichtung ist es vorteilhaft, wenn die Welle des Rührwerks am Boden des Substratbehälters abgestützt ist, insbesondere, wenn die Abstützung der Welle am Boden des Substratbehälters mit Hilfe eines Lagerbocks erfolgt.

Günstig gestaltet ist eine Vorrichtung auch dann, wenn die Welle des Rührwerks die Wand des Substratbehälters oder dessen Abdeckung durchdringt.

Bei einer Vorrichtung ist es günstig, wenn der Substratbehälter als Fermentationsbehälter, Nachgärbehälter, Endlager oder dergleichen ausgebildet ist.

Eine Vorrichtung ist dann vorteilhaft, wenn der Substratbehälter eine gasdichte Abdeckung aufweist.

Eine Vorrichtung kann vorteilhaft gestaltet werden, wenn die umlaufende Wand des Substratbehälters in der Draufsicht Kreisringförmig ist.

Mit Hilfe eines Ausführungsbeispiels soll die Erfindung anhand der Zeichnungen noch näher erläutert werden.

Es zeigt
Figur 1 einen offenen Substratbehälter einer Biogasanlage mit Teildecke und erfindungsgemäßem Rührwerk;
Figur 2 ein Rührwerk in Detaildarstellung;
Figur 3 eine Einbau-Variante des Rührwerks gemäß der Erfindung und
Figur 4 eine Einzelheit zur Veranschaulichung der Rotationskreise

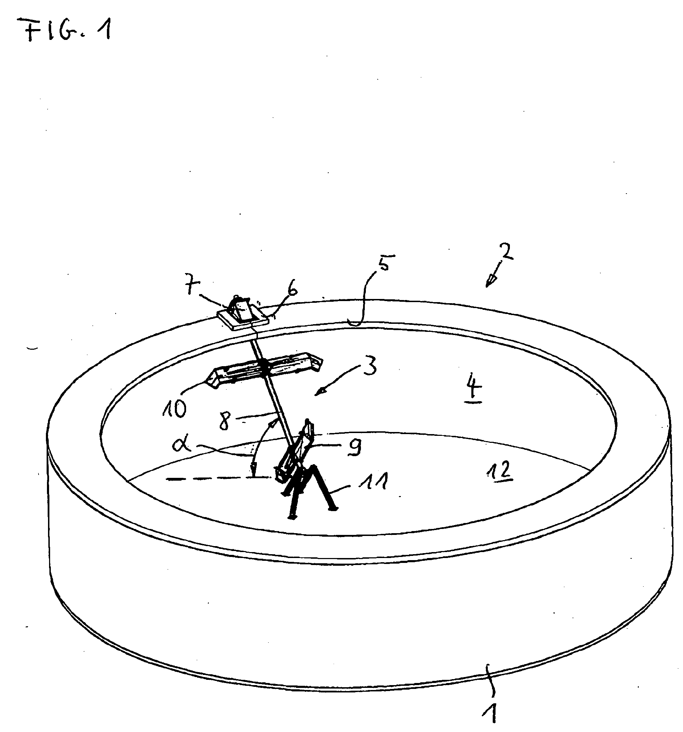

In Figur 1 ist ein offener Substratbehälter gezeigt, der in der folgenden Beschreibung als Fermentationsbehälter 1 einer Biogasanlage 2 bezeichnet wird. Der nicht dargestellte Inhalt soll von einem Rührwerk 3 durchgemischt werden, welches bei diesem Ausführungsbeispiel in einem zylindrischen Fermentationsbehälters 1 angeordnet sind. Eine Wand 4 des Fermentationsbehälters 1 ist vorzugsweise thermisch isoliert und vorzugsweise aus Beton gefertigt. Der obere Begrenzungsrand 5 des Fermentationsbehälters 1 wird von einem Kreisring aus Beton gebildet. Der Betonrand 5 weist eine Verstärkungsplatte 6 auf, mittels der ein Antriebsmotor 7 für eine Rührwelle 8 an dem Fermentationsbehälter 1 gelagert ist. Die Rührwelle 8, ragt unter einem von der Vertikalen und Horizontalen abweichenden Einbauwinkel α in den Fermentationsbehälter 1 hinein und trägt zwei Rührschaufeln 9 und 10, welche längs der Rührwelle 8 an dieser montiert sind. Die Rührschaufeln 9 und 10 sind jeweils in ihrer Längserstreckung zueinander um 90° auf der Rührwelle 8 versetzt angeordnet und weisen entlang der Achse der Rührwelle 8 einen Abstand zueinander auf. An ihrem dem Antriebsmotor 7 abgewandten Ende ist die Welle 8 in einem Lagerbock 11 abgestützt, der sich am Boden 12 des Fermentationsbehälters 1 befindet.

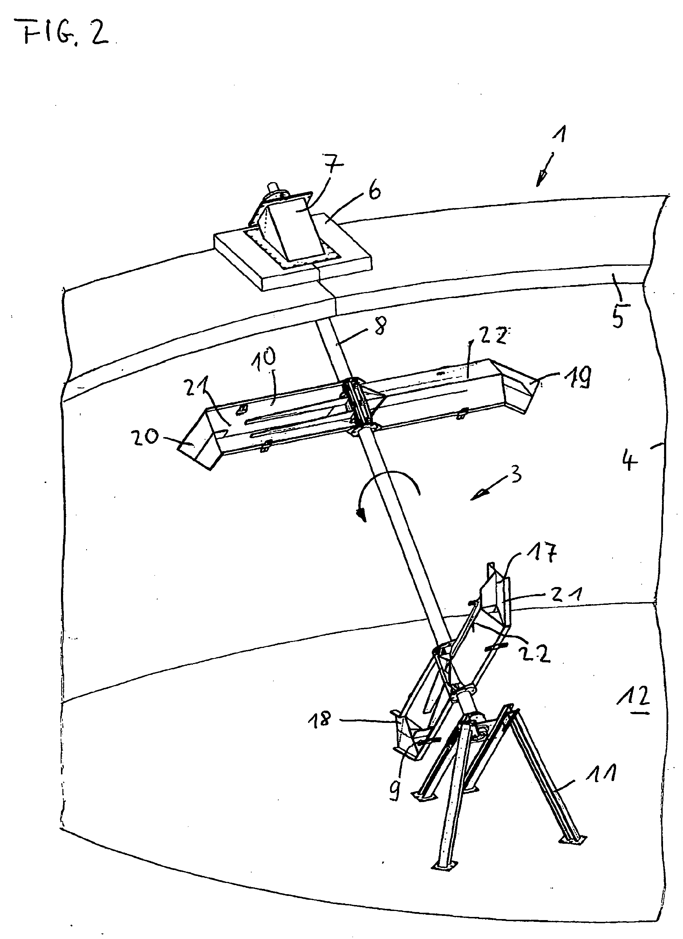

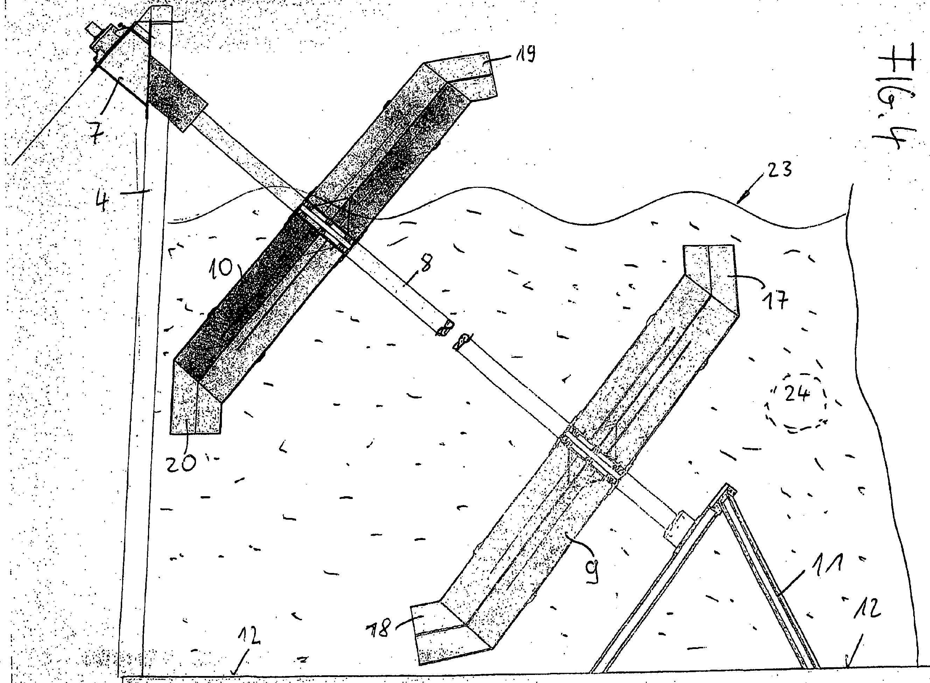

In Figur 2 ist ein Rührwerk 3 in detaillierter Darstellung gezeigt. Um Wiederholungen zu vermeiden sind gleiche oder gleich wirkende Bauteile mit den gleichen Bezugszeichen versehen, wie in Figur 1, auf die ausdrücklich Bezug genommen wird. Mit Hilfe des Antriebsmotors 7 wird die welle 8 in Rotation versetzt und die Rührschaufeln 9 und 10 versetzen das im Fermentationsbehälter 1 befindliche Substrat (hier nicht dargestellt) in Bewegung. Je nach Füllstand des Fermentationsbehälters 1 tauchen die Rührschaufeln 9 und 10 dabei mehr oder weniger in das Substrat ein. Gegenüber dem aus dem Stand der Technik bekannten Propeller-Rührwerken wird das Substrat nicht nur örtlich am Propeller verwirbelt, sondern die Gesamtheit des Substrats 24 wird durch die Rührschaufeln 9 und 10 innerhalb des Fermentationsbehälters in Rotation versetzt, wie in Figur 4 noch veranschaulicht werden wird.

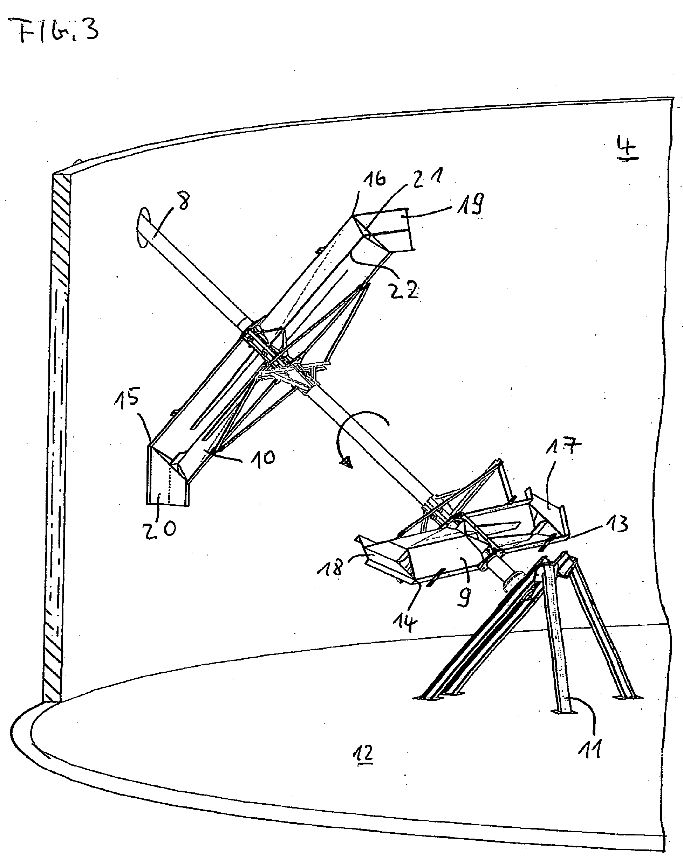

In Figur 3 ist ein Rührwerk 3 in einer Variante dargestellt, bei der die Welle 8 durch die Wand 4 des Fermentationsbehälters 1 hindurch greift. Die Position des Antriebsmotors 7 liegt hier außerhalb der Wand 4 des Fermentationsbehälters 1, so dass er in der Darstellung nicht zu erkennen ist. Gegenüber der Darstellung in Figur 2 sind die Rührschaufeln 9 und 10 etwas verdreht und die Wand 4 ist geschnitten. Sowohl in Figur 3 als auch in Figur 2 ist ersichtlich, dass die Rührschaufeln 9 und 10 an ihren Endbereichen 13, 14, 15 und 16 Abwinklungen 17, 18, 19 und 20 aufweisen. Die Rührschaufeln 9 und 10 sind bevorzugt aus Stahlblech hergestellt und prismatisch abgekantet, so dass sich in Rührrichtung - die der Drehrichtung der Welle 8 entspricht - jeweils ein prismatisches Profil 21 mit einer Schiffsbugartige Schneide 22 ergibt, die beim Rühren im Substrat die Kräfte gering hält. Die Abwinklungen 17, 18, 19 und 20 optimieren die Form der Rührschaufeln 9 und 10 dahingehend, dass die wirksamen Flächen der Rührschaufeln 9 und 10 möglichst nahe an die Randbereiche des Fermentationsbehälters 1 heranreichen, wobei sie in diesen Bereichen ihre Rührwirkung beibehalten, weil der Schaufeleffekt stärker ist, als bei einem Propellerartig rund auslaufenden Ende der Rührschaufeln 9 und 10. Dies gilt sowohl für die Bereiche der Wand 4 als auch für den Boden 12 des Fermentationsbehälters 1.

In Figur 4 ist schematisch das eingebaute Rührwerk 3 gezeigt, wie es aus Figur 3 entnommen sein kann, jedoch ist die Welle 8 unterbrochen, um beide Rührschaufeln 9 und 10 in der gleichen Ansicht sichtbar zeigen zu können. Diese Darstellung erleichtert das Verständnis für den Gegenstand der Erfindung, da sie zeigt, dass die Rotationskreise der Rührschaufeln 9 und 10 sich in Bezug auf das Flüssigkeitsniveau 23 des Substrats 24 im Substratbehälter 1 überlagern. Damit ist gemeint, dass bei steigendem Flüssigkeitsniveau 23 die äußere und weiter oben rotierende Rührschaufel 10 bereits in das Substrat 24 eintaucht, bevor die innere und weiten unten rotierende Rührschaufel 9 vollständig vom Substrat 24 bedeckt ist und sich unterhalb des Flüssigkeitsniveaus 23 befindet.

Diese Merkmale bewirken, dass das Substrat 24 innerhalb des Fermentationsbehälters 1 zu einer Bewegung angeregt wird, die als rotierende Woge bezeichnet werden kann. Bei niedrigem Flüssigkeitsniveau 23 taucht nur der bodennahe Teil der Rührschaufel 9 in das Substrat 24 ein und versetzt es in eine Bewegung, die durch die zylindrische Wand 4 des Fermentationsbehälters 1 zu einer Rotation des Substrats 24 führt. Bei steigendem Flüssigkeitsniveau 23 taucht die gesamte Rührschaufel 9 in das Substrat 24 ein und der oberhalb der Welle 8 befindliche Teil des Rührflügels 9 bewirkt eine gewisse Kompensation des rotatorischen Moments. Beim Gegenstand der Erfindung ist bei diesem Füllstand des Fermentationsbehälters 1 jedoch bereits die zweite Rührschaufel 10 in Aktion und verstärkt ihrerseits das Rotationsmoment im Substrat 24, insbesondere auch dadurch, weil die Rührschaufel 10 sich weiter weg vom Zentrum des Fermentationsbehälter 1 befindet und deshalb das resultierende Drehmoment stärker ist, als bei der ersten Rührschaufel 9, die sich näher am Zentrum des Fermentationsbehälters 1 befindet. Um diesen Sachverhalt begrifflich zu fassen, wurde dieses Merkmal mit dem Begriff der sich in Bezug auf das Flüssigkeitsniveau 23 des Substratsbehälters 1 überlagernden Rotationskreise der Rührschaufeln 9 und 10 bezeichnet.

Bei der erfindungsgemäßen Vorrichtung sind die Länge der Rührschaufeln 9 und 10, ihre axiale Position auf der Welle 8 und der Einbauwinkel der Welle 8 im Fermentationsbehälter 1 also so gewählt, dass sich ihre Rotationskreise in Bezug auf das Flüssigkeitsniveau 23 des Fermentationsbehälters 1 überlagern und durch diese Maßnahme die Rotation der Rührschaufeln 9 und 10 eine rotierende Woge des Substrats 24 im Fermentationsbehälter 1 erzeugen.

### Bezugszeichenliste

- 1: Fermentationsbehälter (Substratbehälter)
- 2: Biogasanlage
- 3: Rührwerk
- 4: Wand
- 5: Oberer Begrenzungsrand
- 6: Verstärkungsplatte
- 7: Antriebsmotor
- 8: Rührwelle
- 9: Rührschaufel
- 10: Rührschaufel
- 11: Lagerbock
- 12: Boden
- 13: Endbereich
- 14: Endbereich
- 15: Endbereich
- 16: Endbereich
- 17: Abwinklung
- 18: Abwinklung
- 19: Abwinklung
- 20: Abwinklung
- 21: Profil
- 22: Schneide
- 23: Flüssigkeitsniveau
- 24: Substrat

## Patentansprüche

1. Vorrichtung zum Durchmischen des Inhalts von Substratbehältern (1) mittels eines Rührwerks (3), bei der der Substratbehälter (1) zumindest eine umlaufende Wand (4) und einen Boden (12) umfasst, wobei sich die umlaufende Wand (4) an einer vertikalen Hauptachse orientiert, und das Rührwerk (3) eine Welle (8) umfasst, die unter einem von der Vertikalen und Horizontalen abweichenden Einbauwinkel (α) in den Substratbehälter (1) hineinragt, bei der das Rührwerk (3) wenigstens zwei Rührschaufeln (9, 10) umfasst, die sich jeweils radial beidseits der Welle (8) erstrecken und zueinander verdreht sowie in Längserstreckung der Welle (8) voneinander beabstandet auf der Welle (8) angeordnet sind, wobei die Länge der Rührschaufeln (9, 10), ihre axiale Position und der Einbauwinkel (α) der Welle (8) so gewählt sind, dass sich ihre Rotationskreise in Bezug auf das Flüssigkeitsniveau (23) im Substratsbehälter (1) überlagern, **dadurch gekennzeichnet, dass** die Rührschaufeln (9, 10) bis in ihre Endbereiche (13, 14, 15, 16) ein sich radial zur Welle (8) erstreckendes Rechteckprofil (21) aufweisen, die Endbereiche (13, 14, 15, 16) gegenüber der Erstreckungsrichtung des Profils (21) jedoch Abwinklungen (17, 18, 19, 20) aufweisen.

2. Vorrichtung nach Anspruch 1, **dadurch gekennzeichnet, dass** die Rührschaufeln (9, 10) ein prismatisches Profil (21) mit Schiffsbugartiger Schneide (22) aufweisen.

3. Vorrichtung nach Anspruch 1, **dadurch gekennzeichnet, dass** die Welle (8) des Rührwerks (3) am Boden (12) des Substratbehälters (1) abgestützt ist.

4. Vorrichtung nach Anspruch 3, **dadurch gekennzeichnet, dass** die Abstützung der Welle (8) am Boden (12) des Substratbehälters (1) mit Hilfe eines Lagerbocks (11) erfolgt.

5. Vorrichtung nach Anspruch 1, **dadurch gekennzeichnet, dass** die Welle (8) des Rührwerks (3) die Wand (4) des Substratbehälters (1) durchdringt.

6. Vorrichtung nach Anspruch 1, **dadurch gekennzeichnet, dass** die Welle (8) des Rührwerks (3) die Abdeckung (5) des Substratbehälters (1) durchdringt

7. Vorrichtung nach Anspruch 1, **dadurch gekennzeichnet, dass** der Substratbehälter (1) als Fermentationsbehälter, Nachgärbehälter, Endlager oder dergleichen ausgebildet ist.

8. Vorrichtung nach Anspruch 1, **dadurch gekennzeichnet, dass** der Substratbehälter (1) eine gasdichte Abdeckung aufweist.

9. Vorrichtung nach Anspruch 1, **dadurch gekennzeichnet, dass** die umlaufende Wand (4) des Substratbehälters (1) in der Draufsicht Kreisringförmig ist.

## Claims

1. Mixing device for contents of substrate container (1) fitted with an agitator (3) in which the substrate container (1) consists of at least one peripheral wall (4) and bottom (12), in which the peripheral wall (4) is oriented alongside a main vertical axis, and the agitator device (3) consists of a shaft (8) projecting at a different angle to the one of the vertical and horizontal mounting (α) in the substrate container (1), in which the agitator (3) consists of at least two agitating vanes (9,10), which in each case, extend radially along both sides of the shaft (8), longitudinally along the shaft (8), spaced out along the shaft (8), where the length of the agitator vanes (9,10), their axial position and the angle of mounting (α) of the shaft (8) ensure that they rotate at the level of liquid (23) in the substrate container (1), **characterised by** vanes (9,10) at the ends (13,14,15,16) which are fitted radially to the shaft (8) by lengthening the rectangular profile (21), at the ends (13,14,15,16) along the length of the profile (21) rather than at inclined parts (17,18,19,20).

2. Device according to claim 1, **characterised by** the prismatic profile (21) of blades (9,10), with a sharp cutting edge (22).

3. Device according to claim 1, **characterised by** the agitator (3) shaft (8) which is supported by the bottom (12) of the substrate container (1).

4. Device according to claim 3, **characterised by** the shaft (8) support rod at the bottom (12) of the substrate container (1) with holes (11).

5. Device according to claim 1, **characterised by** the agitator (3) shaft (8) which crosses the wall (4) of the substrate container (1).

6. Device according to claim 1, **characterised by** the agitator (3) shaft (8) crosses the cover (5) of the substrate container (1).

7. Device according to claim 1, **characterised by** the substrate container (1) which is designed as a fermentation vessel, a fermentation container, a deposit tank or similar.

8. Device according to claim 1, **characterised by** the the substrate container (1) which is equipped with a gas tight lid.

9. Device according to claim 1, **characterised by** the peripheral wall (4) of the substrate container (1) which is ring shaped when viewed from above.

## Revendications

1. Dispositif de mélange du contenu du récipients des substrats (1) au moyen d'un agitateur (3), dans lequel le récipient des substrats (1) comprend au moins une paroi périphérique (4) et un fond (12), dans lequel la paroi périphérique (4) s'oriente par rapport à un axe principal vertical, et le dispositif d'agitation (3) comprend un arbre (8) qui fait saillie avec un angle différent de l'angle de montage vertical et horizontal (α) dans le récipient de substrat (1), dans lequel l'agitateur (3) comprend au moins deux pales d'agitation (9, 10) qui, dans chaque cas, radialement des deux côtés de l'arbre (8) s'étendent et qui sont disposées l'une par rapport à l'autre ainsi que longitudinalement par rapport à l'arbre (8), espacés sur l'arbre (8), dans lequel la longueur des pales de l'agitateur (9, 10), leur position axiale et l'angle de montage (α) de l'arbre (8) sont tels que leurs cercles de rotation se confondent avec le niveau de liquide (23) dans le récipient des substrats (1), **caractérisé en ce que** les pales (9, 10) au niveau des zones d'extrémité (13, 14, 15, 16) ont une direction radiale par rapport à l'arbre (8) en prolongeant le profil rectangulaire (21), au niveau des extrémités (13, 14, 15, 16) dans le sens de la longueur du profil (21) plutôt qu'au niveau des parties inclinées (17, 18, 19, 20).

2. Dispositif selon la revendication 1, **caractérisé en ce que** les lames (9, 10) présentent un profil prismatique (21) avec un bord de coupe tranchant (22).

3. Dispositif selon la revendication 1, **caractérisé en ce que** l'arbre (8) de l'agitateur (3) est soutenu par fond (12) du récipient des substrats (1).

4. Dispositif selon la revendication 3, **caractérisé en ce que** le support de l'arbre (8) au fond (12) du récipient des substrats (1) est réalisé à l'aide d'un socle (11).

5. Dispositif selon la revendication 1, **caractérisé en ce que** l'arbre (8) de l'agitateur (3) traverse la paroi (4) du récipient des substrats (1).

6. Dispositif selon la revendication 1, **caractérisé en ce que** l'arbre (8) de l'agitateur (3) traverse le couvercle (5) du récipient des substrats (1).

7. Dispositif selon la revendication 1, **caractérisé en ce que** le récipient de substrats (1) est conçu comme un bac de fermentation, un conteneur de fermentation, une cuve de dépôt ou similaire.

8. Dispositif selon la revendication 1, **caractérisé en ce que** le récipient de substrats (1) est doté d'un couvercle étanche au gaz.

9. Dispositif selon la revendication 1, **caractérisé en ce que** la paroi périphérique (4) du récipient de substrats (1) est de forme annulaire circulaire vue du dessus.
